# EUROPEAN PATENT APPLICATION

(11) **EP 1 224 862 A1**
(43) Date of publication of application: **24.07.2002**
(21) Application number: 00964721.5
(22) Date of filing: 06.10.2000
(51) Int. Cl.: A01K 67/027, A61P 13/12, A61K 45/00, C12N 15/12, C12N 15/85, G01N 33/15, G01N 33/50

(54) **MODEL ANIMAL OF MESANGIAL CELL PROLIFERATIVE NEPHRITIS**

(30) Priority: 06.10.1999 JP 28573699
(71) Applicant: Kurokawa, Kiyoshi, Sinjuku-ku, Tokyo 162-0061 (JP); Miyata, Toshio, Isehara-shi, Kanagawa 259-1132 (JP)
(72) Inventor: MIYATA, Toshio, Isehara-shi, Kanagawa 259-1132 (JP)
(74) Representative: Grünecker, Kinkeldey, Stockmair & Schwanhäusser Anwaltssozietät
(86) International application number: JP0006988
(87) International publication number: WO0124628

(57) **Abstract**

An animal model for mesangial proliferative nephritis was provided using megsin transgenic animals. Transgenic mice constructed by using a megsin gene exhibits typical symptoms of mesangial proliferative nephritis associated with the expansion of the mesangial matrix, the deposition of an immune complexes, and an increase in mesangial cells. This animal model for mesangial proliferative nephritis is useful in analyzing the pathological conditions of chronic glomerular diseases and the screening for therapeutic agents.

## Description

### Technical Field

This invention relates to a transgenic animal model for mesangial proliferative nephritis.

### Background Art

Mesangial cells play a pivotal role in maintaining the structure and function of a glomerulus, and also have a central pathophysiological meaning in each type of nephritis. For example, proliferation of mesangial cells and accumulation of extracellular mesangial matrix are important pathological findings that indicate glomerulosclerosis in patients suffering from various glomerular diseases such as chronic glomerular nephritis and diabetic nephropathy.

Animal models are valuable tools when studying the pathogenesis of diseases. For instance, transgenic mice introduced with the c-myc oncogene (US Patent No. 5087571) made a significant contribution to the understanding of the pathogenesis of leukemia as a leukemia animal model. If an animal model that exhibits symptoms of mesangial cell proliferation is made, it would become a model for various glomerular disorders, but in reality, there has been no report on such a model.

Chronic glomerulonephritis is the major cause of progressive glomerular disorders in humans that lead eventually to renal failure. Thus, elucidation of the causes of the disease using an animal model for this disease has been anticipated. Preparing an animal model for mesangial proliferative nephritis is helpful for understanding biological characteristics of mesangial cells and the causes of diseases relating to mesangial cells, and in turn, for searching for or testing therapeutic agents for diseases relating to mesangial cells.

Thy1 antigen is known as a marker for mesangial cells in rats. However, this gene is not specific to mesangial cells and is not expressed in human mesangial cells (Miyata T. et al., Immunology, 1989, 67: 531-533; and Miyata T. et al., Immunology, 1990, 69: 391-395). Mesangial cells are known to express α smooth muscle actin when activated, but this gene is also not specific to mesangial cells. Thus, it is unlikely that generation of transgenic animals based on these genes would provide an animal model for mesangial proliferative nephritis.

Generally, it has been difficult to generate a renal disease model in mice. For instance, administration of an antibody against glomerular basement membrane (anti-GBM antibody) has been utilized to generate a mouse model for nephritis. In this model, however, there are some problems such as that the symptoms tend to vary among individuals, and besides, it is difficult to obtain significant symptoms compared to a similar model in rat.

Recently, Johnson et al., reported generation of an animal model for renal disease by administrating an antibody against the whole glomerulus. While their model develops severe glomerular dysfunctions that result in the formation of glomerular crescents, the disorder is considered to represent only rapidly progressive glomerulonephritis in humans (Ophascharoensuk et al., Kidney Int. 54: 416 (1998)). Thus, an animal model that develops a disorder similar to human chronic glomerulonephritis was desired.

To answer such needs, genetic engineering technology is being recently utilized to generate animal models. For instance, it has been reported that transgenic animal produced using SV40 (MacKay et al., Kidney Int. 32: 827 (1987)) or growth hormones (Doi et al., Am. J. Pathol. 137: 541 (1990)) develop renal disease. However, the major disorder in these models is glomerulosclerosis, and a change in proliferation of glomerular cells is scarcely found. Transgenic mice into which interleukin-6 gene has been introduced are known to develop renal failure, but the major disorders are glomerulosclerosis and tubulointerstitial damage(Fattori et al., Blood 83: 2570 (1994)). Transgenic mice into which renin and angiotensin genes have been introduced (Tsukuba hypertensive mice) are known to develop glomerulosclerosis (Shimokama et al., Virchows Arch. 432: 169 (1998)). However, the disorder is a result of hypertension, and thus, it can also not to become an animal model for glomerular disorders in humans. Thus, there is no report of transgenic animals that would serve as a model for human glomerular disorders associated with proliferation of mesangial cells and expansion of mesangial matrix. Furthermore, the renal disorders in conventional transgenic mice are often found by chance as a result of an unexpected influence of a gene that had been introduced for another purpose. Thus, there has been no report that indicates which gene(s) should be used to generate an animal model for human glomerular diseases.

The present inventor has previously reported megsin as a protein that is expressed specifically in mesangial cells (J. Clin., Invest, 1998 Aug 15, 102: 4, 828-36). Nevertheless, it remains unclear whether or not introduction of a DNA encoding a megsin protein can generate an animal model for mesangial proliferative nephritis.

### Disclosure of the Invention

An objective of this invention is to provide an animal model for mesangial proliferative nephritis and a method for preparing the animal model. It also provides a method of screening for a therapeutic agent with the use of the animal model for mesangial proliferative nephritis obtained by the present invention.

In order to solve the above-described issues, the present inventor focused on megsin and a DNA encoding it. Megsin is a protein encoded by a gene specifically expressed in mesangial cells (J. Clin. Invest. 120 (4): 828-836 (Aug. 15, 1998)). The present inventor identified the structure of human megsin and its mouse and rat homologues, and filed patent applications for their discoveries in WO 99/15652. However, it remained unclear how mesangial cells are influenced by an overexpression of megsin. The present inventor prepared transgenic mice carrying a DNA encoding a megsin, and confirmed that these transgenic mice develop a typical mesangial proliferative nephritis. In the glomerular tissues of transgenic mice at about 35-40 weeks of age, there were increased proliferation of mainly mesangial cells, expansion of mesangial matrix, and increased deposition of immune complexes comprising immunoglobulin and complement, and the mice were observed to have developed segmental sclerosis. Based on the results, the present inventor discovered that it is possible to prepare a useful transgenic animal model for mesangial proliferative nephritis by introducing a DNA encoding a megsin, and accomplished this invention. Thus, this invention relates to an animal model for mesangial proliferative nephritis, a method for preparing it and its use as follows:
(1) an animal model for mesangial proliferative nephritis, wherein the animal model is prepared using a non-human transgenic animal that expresses an exogenous DNA encoding human megsin, or a functionally equivalent protein thereof, in its renal mesangial cells;
(2) the animal model for mesangial proliferative nephritis according to (1), wherein the DNA encoding human megsin comprises the coding region of the nucleotide sequence of SEQ ID NO: 1;
(3) the animal model for mesangial proliferative nephritis according to (1), wherein the non-human animal is a mouse;
(4) a transgene for generating an animal model for mesangial proliferative nephritis, comprising a DNA encoding human megsin, or a functionally equivalent protein thereof, and a promoter capable of induces expressing expression of the DNA in renal mesangial cells of the animal;
(5) a method for preparing an animal model for mesangial proliferative nephritis, comprising the steps of:
   (a) introducing the transgene according to (4) into a fertilized egg of an animal, and,
   (b) selecting the transgenic animal that carries the introduced exogenous gene;
(6) the method for preparing an animal model of mesangial proliferative nephritis according to (5), further comprising the following steps of:
   (c) crossing the animal selected in step (b) with a wild-type animal to obtain F1 progenies comprising exogenous heterozygous genes, and,
   (d) crossing the F1 progenies obtained in step(c) with each other to obtain F2 progenies comprising exogenous homozygous genes;
(7) a method of predicting the therapeutic effect of a candidate compound on mesangial proliferative nephritis, comprising the following steps of:
   (a) administrating a candidate compound to the animal model of mesangial proliferative nephritis according to (1), and,
   (b) observing a change in a symptom of nephritis in the animal model;
(8) a method of screening for a therapeutic compound for mesangial proliferative nephritis, comprising the following steps of:
   (a) administrating a candidate compound to the animal model for mesangial proliferative nephritis according to (1),
   (b) observing a change in a symptom of nephritis in the animal model, and,
   (c) selecting the candidate compound that ameliorates the symptom of mesangial proliferative nephritis; and,
(9) a pharmaceutical composition for treatment and/or prevention of mesangial proliferative nephritis, wherein the pharmaceutical composition comprises a compound obtained by the method of screening according to (8) as the active ingredient.

The present invention also relates to a use of a transgene for generating an animal model for mesangial proliferative nephritis, the gene comprising a DNA encoding a human megsin or a functionally equivalent protein thereof and a promoter capable of expressing the DNA in renal mesangial cells in the animal model.

The animal model for mesangial proliferative nephritis according to this invention may be obtained by the standard method for producing transgenic animals. Herein, mesangial proliferative nephritis means a renal disorder associated with proliferation of renal mesangial cells and expansion of mesangial matrix. Such renal disorders include IgA nephropathy, membranoproliferative glomerulonephritis, SLE (systemic lupus erythematosus) nephropathy, diabetic nephropathy, cryoglobulin nephropathy, etc. For example, a transgenic animal may be generated according to the method described in "Hassei Kougaku Jikken Manual (Protocols in developmental biology)" (Nomura T. edit. Katsuki M. edit. Kodansha (1989)), or "Shin Seikagaku Jikken Kouza, Doubutsu Jikkenhou (New Protocols in Biochemistry, Animal Experiments)." (Edited by Japanese Biochemical Society , Tokyo Kagaku Doujin (1991)). The standard protocol for generating a transgenic animal is described below.

Herein, human megsin is defined as a protein encoded by a DNA comprising the nucleotide sequence according to SEQ ID NO: 1. Its deduced amino acid sequence is shown in SEQ ID NO: 2. The transgenic animal of this invention may be introduced with a DNA encoding not only a human megsin, but also a functionally equivalent protein thereof. Such a protein may be a megsin homologue in other species. The present inventor identified the structure of megsin homologue in the rat and mouse. The nucleotide sequence of rat megsin and its amino acid sequence are shown in SEQ ID NO: 3 and 4, respectively. The nucleotide sequence of mouse megsin and its amino acid sequence are shown in SEQ ID NO: 5 and 6, respectively.

In general, an eukaryotic gene often shows polymorphism, like the human interferon gene. Even though one or more aminoacids in an amino acid sequence may be replaced due to this polymorphism, generally, the protein activity will be maintained. In general, activities of proteins can be often maintained even if one or more amino acids are modified. Therefore, a gene encoding a protein comprising an amino acid sequence in which any one of the amino acid sequences of SEQ ID NO: 2, 4, or 6 has been artificially modified can be used in this invention, as long as the protein causes mesangial proliferative nephritis. Hereafter, human megsin, its homologues in rat and mouse, and proteins functionally equivalent to human megsin will be collectively referred to as "megsins". Additionally, if a DNA encoding a mouse megsin is artificially introduced into a mouse, DNA derived from the mouse is exogenous DNA. However, a DNA encoding a human megsin is advantageous when screening for a compound that would be useful as a therapeutic agent in humans using the transgenic animal of the present invention as a model for mesangial proliferative nephritis. By doing so, the effect a compound has on human megsin within the transgenic animal can be predicted more appropriately.

Codons for amino acids are well known, and can be arbitrarily selected. For example, codons can be determined by following the standard method by, for example, considering the frequency of use of codons in hosts used (Grantham, R. et al., Nucleic Acids Res. 9, r43, 1981). Therefore, the present invention includes DNAs appropriately modified by degeneration of codons. It is possible to partially change the codons of these nucleotide sequences according to standard methods such as site-specific mutagenesis (Mark, D. F. et al., Proc. Natl. Acad. Sci. U.S.A. 1984, 81: 5662).

A DNA that hybridizes with a DNA comprising the nucleotide sequence according to any one of SEQ ID NO: 1, 3, or 5 and encodes a protein that causes mesangial proliferative nephritis, can be included in the DNA of the present invention. A sequence capable of hybridizing with a specific sequence under stringent conditions is thought to have an activity similar to a protein encoded by the specific sequence. Stringent conditions may be normally achieved in "1x SSC, 0.1% SDS at 37°C", more stringent conditions may be achieved in "0.5x SSC, 0.1% SDS at 42°C", and even more stringent conditions may be achieved in "0.1x SSC, 0.1% SDS at 55°C". A partial fragment of a DNA encoding a megsin of this invention may also be used, as long as it causes mesangial proliferative nephritis in a transgenic animal.

The DNA of this invention encoding a megsin used for producing a transgenic animal may be obtained by a well-known method based on a nucleotide sequence disclosed herein. For instance, a DNA comprising the nucleotide sequence according to SEQ ID NO: 1, 3, or 5 may be used as a probe to screen a cDNA library made from mesangial cells to isolate a cDNA encoding a megsin. Alternatively, primers may be designed based on the nucleotide sequence according to SEQ ID NO: 1, 3, or 5, and used in PCR using the above cDNA library as a template to amplify a DNA encoding a megsin. The amplified products may be cloned according to the well-known methods.

It is advantageous to prepare a DNA encoding a megsin as a transgene construct by ligating the DNA encoding the megsin with a promoter that is capable of expressing the DNA in an animal cell to which this gene is introduced. The transgene construct of this invention may be constructed by using a cloning vector in an appropriate host. The DNA encoding the megsin may be inserted into the cloning vector and a promoter may be cloned into its upstream region. A promoter that can be used may be the chicken β-actin promoter, which is capable of inducing the expression of an exogenous gene in abroad range of vertebrates such as mice and rats.

An enhancer sequence may be additionally inserted as well in order to enhance the expression of the exogenous gene. For instance, the CMV-derived enhancer is known to enhance the expression of an exogenous gene in mammals.

For constructing a construct comprising the above gene, a vector that is equipped with an enhancer and promoter as well as a multicloning site for inserting the exogenous gene in its downstream may be used. Such a vector may be constructed from the pCAGGS vector (Niwa H., Yamanaka K. and Miyazaki J., "Efficient selection for high-expression transfectants with a novel eukaryotic vector", Gene 108: 193-200 (1991)), for instance, according to the method described in the Example. In this vector, the rabbit β-globin terminator sequence is positioned downstream of the multicloning site, and this improves the expression of the inserted exogenous gene.

The transgene construct may be cut out of the vector by digesting with appropriate enzymes, purified sufficiently, and used for producing a transgenic animal. The transgenic animal can be obtained by introducing the construct into an unfertilized or fertilized egg, sperm, or an embryo containing primordial germ cells, etc. The cell into which the construct is introduced may be selected at the stage of embryogenesis in a non-human animal, more specifically, it may be a single cell, or fertilized egg typically before the 8-cell stage. The transgene construct may be introduced by well-known methods such as those utilizing calcium phosphate, electroporation, lipofection, aggregation, microinjection, particle gun, or DEAE-dextran. The resulting transformant may be fused to the above-described embryo to generate the transgenic animal.

The cell introduced with the transgene construct may be any cell from a non-human vertebrate from which a transgenic animal can be produced. Specifically, a cell derived from a mouse, rat, hamster, guinea pig, rabbit, goat, sheep, pig, cow, dog, cat, and such may be used. In the case of mice, fertilized eggs into which a construct can be introduced may be obtained by crossing a female injected with an ovulation inducer with a normal male. In fertilized eggs of mice, a transgene construct is normally introduced into the male pronuclei by microinjection. The cells into which the construct was introduced may be cultured *ex vivo* overnight, and then successfully-introduced cells may be implanted in the oviduct of a surrogate mother to generate transgenic chimeric mice. Pseudopregnant females that were crossed with male mice whose seminal duct had been ablated may be used as surrogate mothers.

The resulting transgenic chimeric animal may be checked for the integration of exogenous gene (a DNA encoding a megsin) into the genome by analyzing the gene in the somatic cells. Then, the chimeric animal may be crossed with a wild-type animal to obtain a F1 progeny. It is preferable to select mice carrying a high copy number because such animals are more likely to develop a clear phenotype. This is because exogenous DNA that is introduced as a construct is usually integrated as a plurality of copies into the same region of the genome in tandem, and the number of copies generally correlates with the level of expression. The orientation of the integrated exogenous gene in the genome of somatic cells may be checked by PCR using primers specific to the construct. The copy number among individual animals can be compared by a dot blot.

The resulting F1 progenies carrying the exogenous gene (a DNA encoding a megsin) in somatic cells are transgenic animals that are heterozygotes, but also are capable of transferring the exogenous gene (a DNA encoding a megsin) to germ cells. Accordingly,by selecting F1 progenies carrying the exogenous gene (a DNA encoding a megsin) in somatic cells, and crossing these F1 progenies with each other, F2 progenies that are homozygotes carrying a homozygous exogenous gene (a DNA encoding a megsin)can be obtained.

The animal model for mesangial proliferative nephritis of this invention may be from any generation of such transgenic animals, as long as it expresses an exogenous DNA encoding a megsin in mesangial cells. For instance, a transgenic animal carrying a heterozygous exogenous DNA encoding a megsin may also be utilized as an animal model for mesangial proliferative nephritis if it expresses the exogenous megsin in mesangial cells.

Herein, the animal model for mesangial proliferative nephritis includes those animals expressing an exogenous DNA encoding a megsin at least in mesangial cells. In other words, the DNA is not necessarily expressed in the kidney specifically. For instance, transgenic mice expressing human megsin systemically can also develop a clear phenotype of mesangial proliferative nephritis as described in the Example.

The fact of whether the transgenic animal shows symptoms of mesangial proliferative nephritis may be verified by using the following indexes. The renal tissues may be stained with PAS, and the number of mesangial cells and the area of the mesangial matrix may be calculated to quantify the severity of proliferative glomerulonephritis. Specifically, the following pathological findings are exemplified as typical findings for the transgenic animal of this invention. Namely, in the renal glomerular tissue of the transgenic animal of this invention at about 35-40 weeks of age, a significant proliferation of cells (mainly mesangial cells) is seen, and an expansion of the mesangial matrix, deposition of immune complexes comprising immunoglobulin and complement, and segmental sclerosis are observed.

The animal model for mesangial proliferative nephritis using the transgenic animal of the present invention develops a typical mesangial proliferative nephritis associated with cell proliferation and expansion of the extracellular matrix in the mesangium and glomerular endothelium in a chronically progressive manner. Additionally, the generation of the model requires fewer procedures and provides a homogenous population in a large quantity compared to models generated by other methods such as injecting a nephritis inducer or surgery. Therefore, the animal model of this invention is useful for the understanding of the pathogenesis and mechanism of mesangial proliferative nephritis, as well as for large-scale screening aimed at developing a therapeutic agent.

The animal model for mesangial proliferative nephritis of this invention may also be utilized to predict the effectiveness of a candidate compound for treating mesangial proliferative nephritis. The method of prediction of this invention comprises the following steps of:
(a) administrating a candidate compound to the animal model of mesangial proliferative nephritis of this invention; and
(b) observing a change in a symptom of nephritis in the animal model.

Also, the animal model for mesangial proliferative nephritis of this invention may be used to screen for a compound for treating mesangial proliferative nephritis. The method of screening of this invention comprises the following steps of:
(a) administrating a candidate compound to the animal model for mesangial proliferative nephritis of this invention;
(b) observing a change in a symptom of nephritis in the animal model; and
(c) selecting the candidate compound that ameliorates the symptom of mesangial proliferative nephritis.

In the method of prediction or screening of this invention, the effectiveness of a candidate compound for ameliorating nephritis symptoms may be evaluated based on the pathological findings of renal tissues, or if a candidate compound is expected to have an activity of regulating the expression level of a megsin, the level of the megsin mRNA or protein in mesangial cells may be examined as well. The results of pathological findings of renal tissues may be quantified as described in the Example, then the levels of nephritis symptoms may be compared. The mRNA expression level may be examined by a well-known method using a probe or primers deigned based on the nucleotide sequence of a DNA encoding a megsin, such as the nucleotide sequence according to SEQ ID NO: 1. The level of protein expression may be examined by an immunoassay using an antibody against a protein comprising the amino acid sequence according to SEQ ID NO: 2.

The effectiveness of a candidate compound as a therapeutic agent can be estimated by comparing observations based on the above indexes before and after administration of the compound. Alternatively, the effectiveness of the compound may be compared by comparing observations among transgenic animals of the same line.

For example, a candidate compound used in the screening of this invention may include natural or synthetic compounds, various organic compounds, natural or synthetic saccharides, proteins, peptides, products of gene libraries, cell extracts, and bacterial or fungal components. It may also include antisense nucleic acids capable of regulating the expression of megsins, and anti-megsin antibodies that could inhibit the activity of megsins. These candidate compounds may be orally or parenterally administered to the animal model for mesangial proliferative nephritis.

The candidate compound selected by the screening of this invention may be further tested for safety and stability, and utilized as the active ingredient of a pharmaceutical composition for the treatment and/or prevention of mesangial proliferative nephritis. The pharmaceutical composition of this invention may be formulated by well-known pharmaceutical manufacturing methods and administered. The compound that is the active ingredient may be directly administered by itself.Alternatively, the compound may be formulated using an appropriate solvent or carrier that is commonly used for formulating a pharmaceutical preparation.

If a DNA can encode the compound, the DNA may be inserted into a vector developed for gene therapy, and gene therapy may be performed. The vector may be administered intraarterially, intravenously, intranasally, intrabronchially, intramuscularly, subcutaneously or orally, or directly applied to the affected area. Although dose may vary depending on the body weight, age, or condition of the patient, or the method of administration, and so on, one skilled in the art can easily determine an appropriate dose.

For instance, the effective dose may be determined by comparing the ameliorating effect of the compound at different doses on a symptom of nephritis in the animal model for mesangial proliferative nephritis of this invention. Then, the dose of the compound may be determined empirically to achieve the effective concentration of the compound in mesangial cells for each administration method. In general dosage forms, dose may be determined as amount per kg body weight by assuming that the active ingredient of the drug is distributed systemically. If the compound has a high delivery rate to the kidney based on pharmacodynamic analysis results using animal models, the dose may be reduced.

The pharmaceutical composition of this invention may be combined with a solvent or carrier taking into account the determined dose and the dosage form. One skilled in the art may usually blend the effective component with such a solvent or carrier so as to achieve the required dose. The dose of the pharmaceutical composition of this invention may be normally 1 µg to 10 mg/kg body weight, and generally 10 µg to 1 mg/kg body weight. If the composition is administered by an injection, dose may be 1/100 of that used for oral administration. Alternatively, dose may be adjusted by applying a special drug design. For instance, the pharmaceutical composition may be mixed with an appropriate carrier and formulated as a sustained release preparation. Such a formulation allows the maintenance of a high concentration in blood so that the dose may be substantially reduced.

Moreover, the animal model for mesangial proliferative nephritis of this invention may be used for the following purposes in addition to the screening for a therapeutic or preventive compound. The animal model may be used as a material for evaluating indicators for life style improvement, including diet control. Also, since it shows symptoms similar to those of human glomerulonephritis, the animal model for mesangial proliferative nephritis may be used to understand the mechanism of human glomerulonephritis as well. Furthermore, it is possible to use the transgenic animal to study the relationship between genetic and environmental factors in the development of glomerulonephritis because the animal develops glomerulonephritis spontaneously at about 35-40 weeks of age. .

### Brief Description of the Drawings

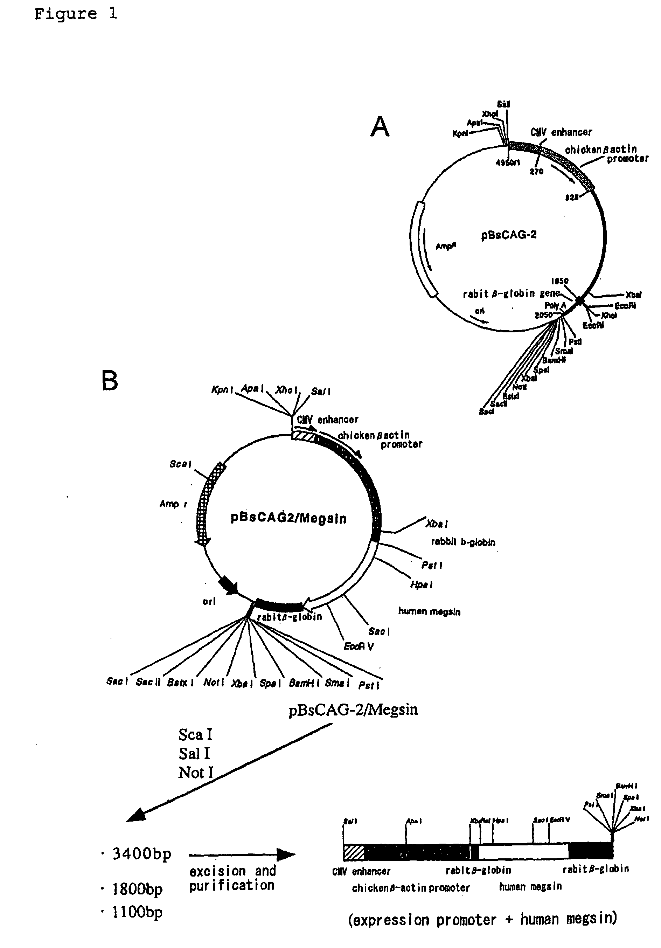

Figure 1 shows a construction scheme for preparing a transgene construct. (A) The structure of pBsCAG-2 vector. (B) The structure of pBsCAG2/Megsin plasmid and a transgene DNA fragment used for microinjection into eggs.

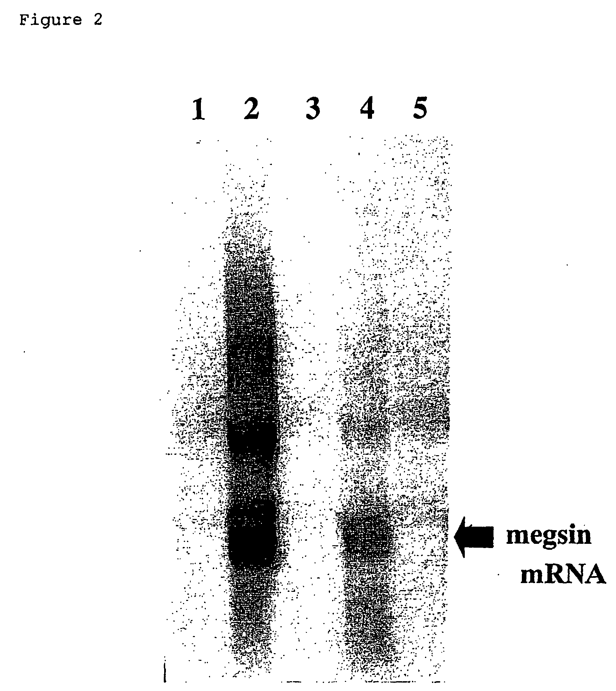

Figure 2 is a photograph showing the result of northern blot analysis of the expression of human megsin gene in renal mesangial cells in transgenic mice. Lane 1 and 3: non-transgenic littermates used in lane 2 and 4, respectively. Lane 2 and 4: transgenic mice. Lane 5: wild type. Mice are from transgenic line A (lane 1 and 2), line B (lane 3 and 4), and wild-type (lane 5).

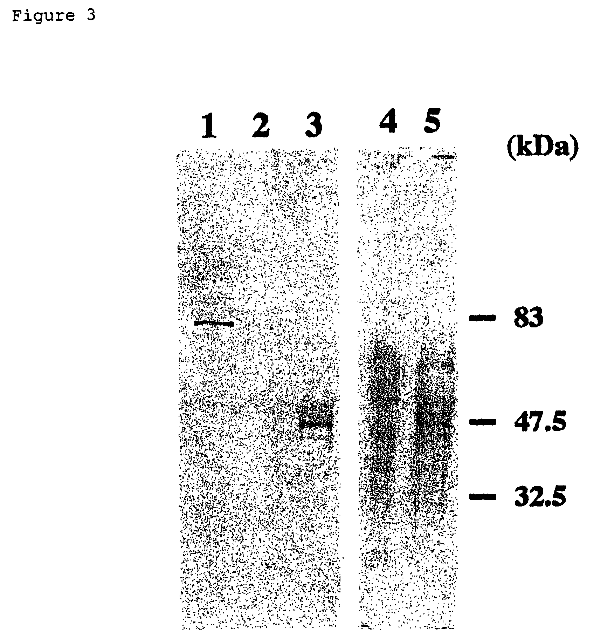

Figure 3 is a photograph showing the result of immunoblot analysis of the expression of human megsin protein in kidneys of transgenic mice using anti-human megsin peptide antibody. Lane 1: recombinant fusion protein between maltose binding protein and megsin (positive control) that was expressed in *E. coli* cells, lane 2 and 4: show results for kidneys from wild-type mice, and lane 3 and 5: show results for kidneys from transgenic mice (line A and B, respectively).

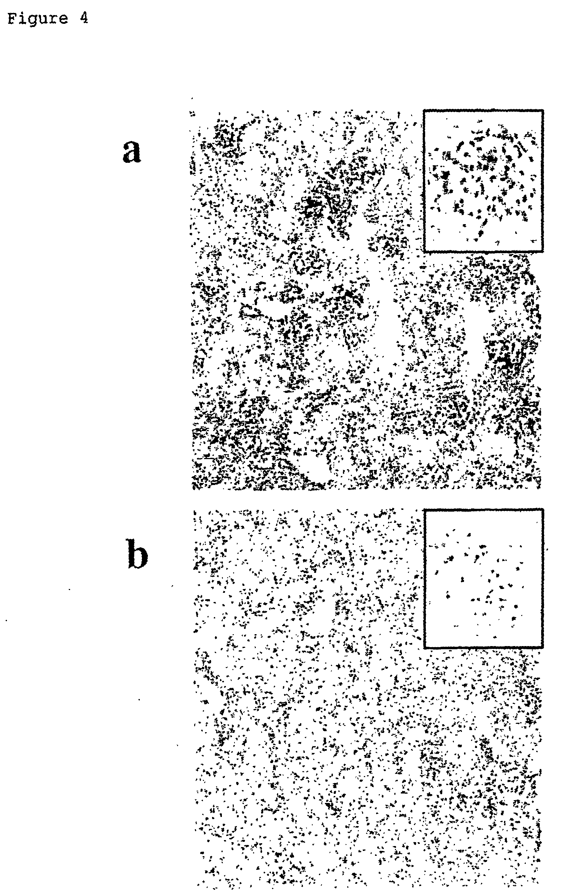

Figure 4 is a photograph showing the result of immunohistochemical detection of human megsin gene product in a kidney of a F1 megsin transgenic mouse (a) and a wild-type mouse (b) using anti-human megsin peptide antibody. Magnification was x 16 (in inserted panels, x 50).

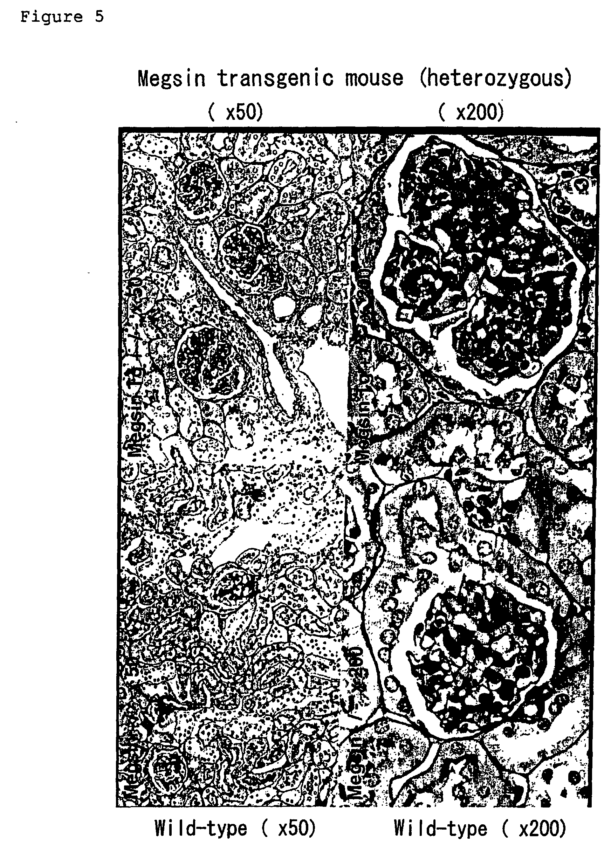

Figure 5 is a photograph showing the result of PAS staining of kidney sections from a normal mouse and from a mouse of the mesangial proliferative nephritis model. Wild type littermate: wild-type mouse. Megsin Tg/+: megsin transgenic mouse (heterozygote).

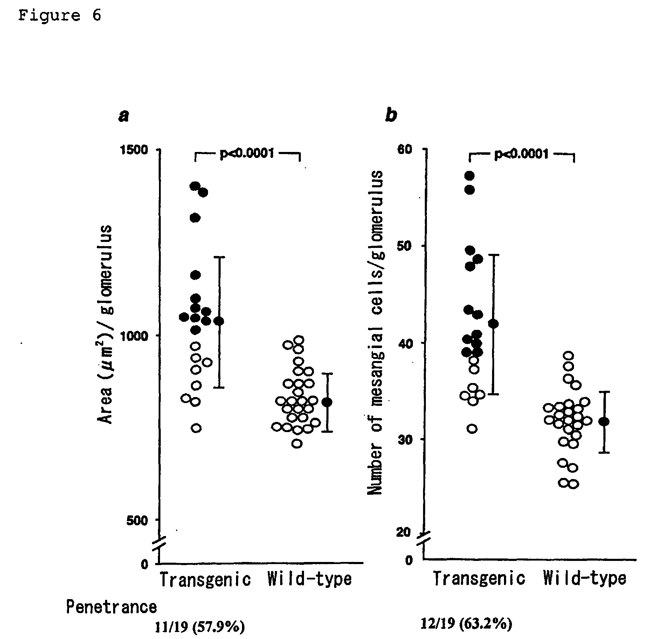

Figure 6 shows the result of glomerular size (a) and numbers of mesangial cells per glomerulus (b) obtained by measuring 25 wild-type mice and 19 megsin transgenic mice. Among the values measured for the transgenic mice, open circles show values that are equal to or less than the standard for wild type mice, mean +2SD. Closed circles represent animals having values above the standard.

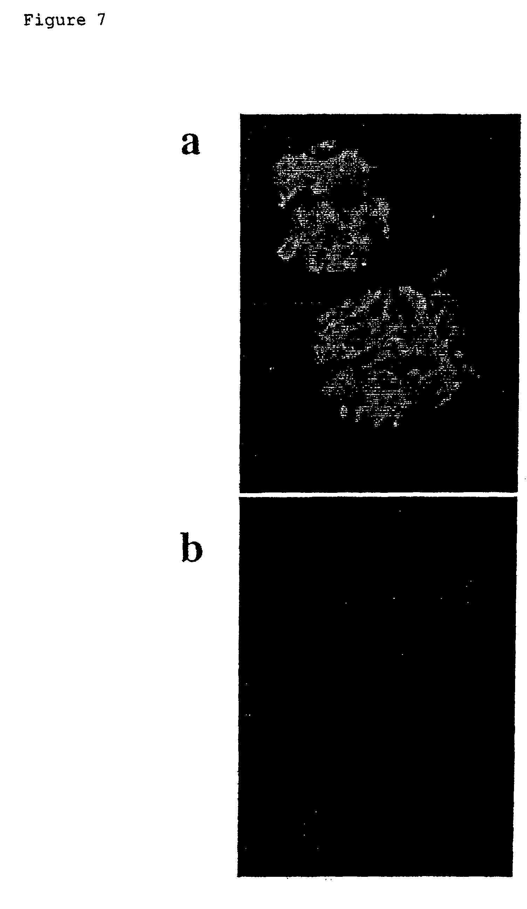

Figure 7 is a photograph showing the result of immunofluorescence analysis of the deposition of immune complexes comprising immunoglobulin (IgA, IgG, and IgM) and complement in glomerular mesangial regions. F1 megsin transgenic mouse (a), and wild type mouse (b) were analyzed at 40 weeks of age. Magnification was x 50.

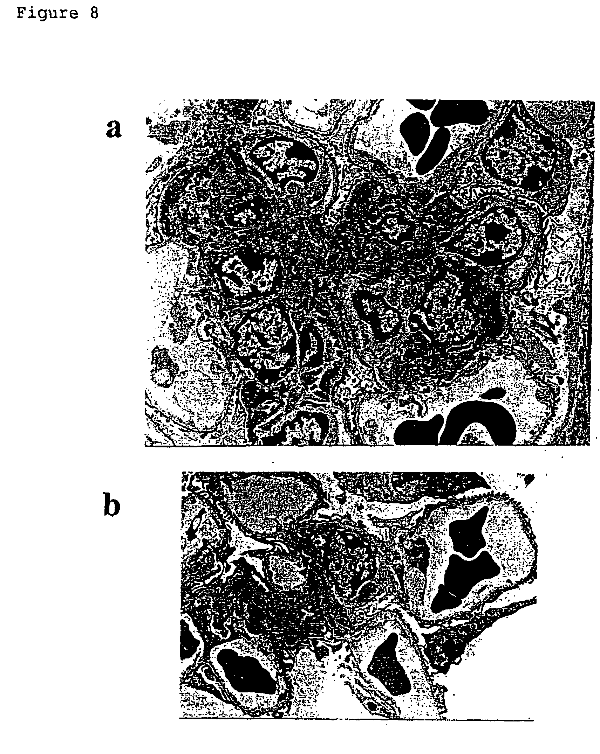

Figure 8 is a photograph showing the result of electron microscopy analysis of the deposition of immune complexes in glomerular mesangial regions of F1 megsin transgenic mouse (a) and in wild type (b) at 40 weeks of age. Magnification was x 2500.

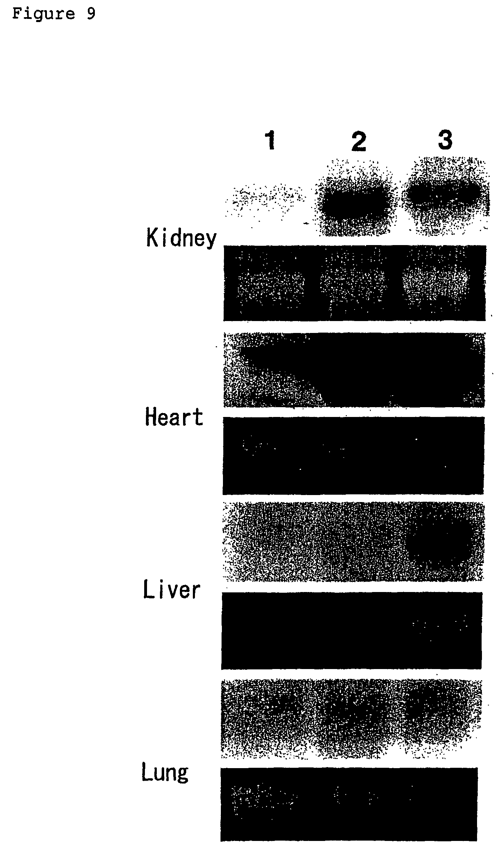

Figure 9 is a photograph showing the result of northern blot analysis of the expression of human megsin mRNA in various tissues of transgenic mice. The result shows expression in kidney, heart, liver, and lung, from top, respectively. In each panel, lane 1: wild type, lane 2: transgenic mice of line A, and lane 3: transgenic mice of line B. For each tissue, top panel shows the result of northern blot, and lower panel shows ethidium bromide staining of RNA after electrophoresis.

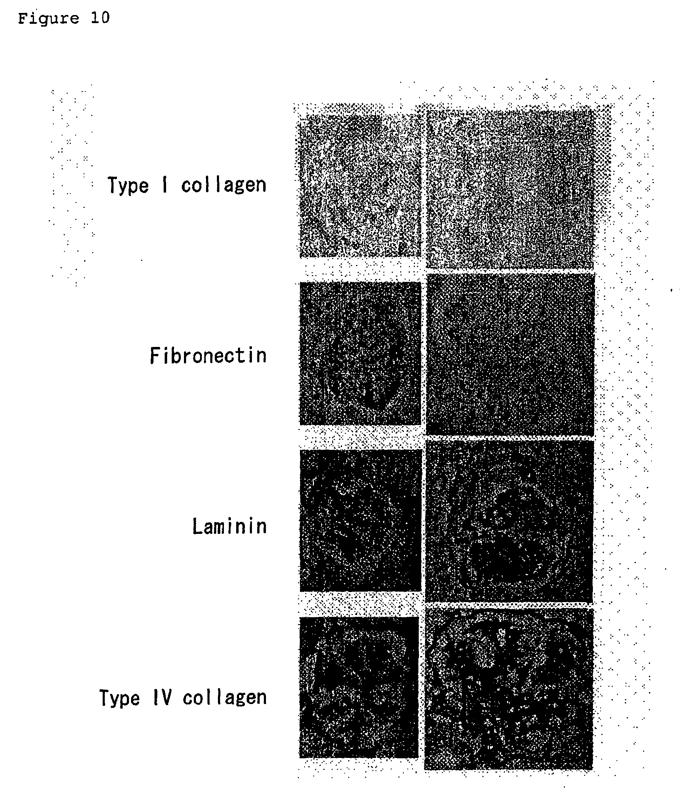

Figure 10 is a photograph showing the result of immunohistochemical analysis of kidneys of transgenic mice and wild type mice. Anti-type I collagen antibody, anti-fibronectin antibody, anti-laminin antibody, and anti-type IV collagen antibody were used, from top, respectively. The right column shows the results with transgenic mice (line A).

### Best Mode for Carrying out the Invention

This invention will be explained in detail below with reference to examples, but it is not to be construed as being limited thereto.

### [Example 1] Construction of transgene

An expression vector carrying a human megsin cDNA under the control of the CAG promoter, a hybrid of the CMV enhancer and the chicken β-actin promoter, was constructed as follows. First, to replace the nucleotide sequence immediately prior to the initiation codon of human megsin with the Kozak sequence (GCCGCC), PCR was performed using the pUC-MEGSIN plasmid containing human megsin cDNA (WO99/15652) as a template with primers (B44F: 5'-ATG GAT CCG CCG CCA TGG CCT CCC TTG CTG CAG CAA ATG CAG AG-3', SEQ ID NO: 7 (sense), and H30-R: 5'-TAT CCT GAG GCA GTG TTA ACA TGA AG-3', SEQ ID NO: 8 (antisense)), and the 5'fragment of human megsin cDNA in which the immediate upstream of the initiation codon has been replaced with the Kozak sequence was amplified. This fragment was inserted into the pUC-MEGSIN that had been digested with BamHI and HpaI so as to remove a fragment of approximately 180 bp containing the initiation codon of megsin cDNA, and a plasmid containing human megsin cDNA following the Kozak sequence was obtained.

The plasmid was used to transform *E. coli* JM109 cells and recombinant clone was obtained. The recombinant clone was digested with BamHI and HindIII to generate a 1.2 kb fragment containing a full length megsin cDNA, which was purified and blunt-ended using the TaKaRa Blunting Kit (TaKaRa). The pBsCAG-2 vector (Kawarabayashi T. et al., Shoji M., Sato M., Sasaki A., Ho L., Eckman C. B., Prada C-M,Younkin S. G., Kobayashi T., Tada N., Matsubara E., Iizuka T., Harigaya Y., Kasai K. and Hirai S., "Accumulation of b-amyloid fibrils in pancreas of transgenic mice." Neurobiol. Aging 17: 215-222 (1996)) was linearized by digesting with EcoRI, blunt-ended as described above, and dephosphorylated by treating with alkaline phosphatase (TaKaRa). The resulting plasmid was ligated with the above 1.2 kb fragment to generate a recombinant plasmid, which was transformed into JM109 cells and cloned. Recombinant plasmid in which the human megsin cDNA was cloned in the same orientation as the chicken β-actin promoter was selected by sequencing, and named as pBsCAG2Megsin (Figure 1B). The pBsCAG-2 vector was constructed by inserting the SalI-PstI fragment of the pCAGGS vector (Niwa H., Yamamura K., and Miyazaki J., "Efficient selection for high-expression transfectants with a novel eukaryotic vector." Gene 108: 193-200 (1991); a mammalian expression vector containing the CMV enhancer), the chicken β-actin promoter, and the rabbit β-globin terminator, into the SalI-PstI site of the pBluescript™ II SK(-) plasmid vector (Stratagene) (Figure 1A).

The pBsCAG2Megsin vector was digested with ScaI, SalI, and NotI, and separated by electrophoresis on an agarose gel, from which a 3.4 kb fragment containing megsin cDNA was recovered and used for the generation of transgenic mice (Figure 1B).

### [Example 2] Generation of transgenic mice

Female mice of 8-20 weeks of age (F1 progenies between C57BL/6 and C3H mice) received PMSG (pregnant mare's serum gonadotropin) intraperitoneally in the evening 3 days before microinjection was performed, and in the evening two days later, also received hCG (human chorionic gonadotropin) in the same manner. Then, a male mouse of 8-20 weeks of age (C57BL/6) was placed in each cage containing a female mouse, and were allowed to copulate. The next morning, female mice were examined for the formation of a vaginal plug, and those with a vaginal plug were sacrificed by dislocating their cervical vertebrae. The oviducts were isolated, and transferred into the Whitten medium supplemented with hyaluronidase. Eggs were removed from the oviduct under a stereoscopic microscope, and fertilized eggs were isolated and washed.

Using a system consisting of an inverted microscope with Nomarski differential contrast attachment and a manipulator, 5 to 30 fertilized eggs were transferred into a drop of growth medium contained in the well of a slide glass. Two pl of DNA solution containing about 2000 copies of the DNA fragment per one fertilized egg prepared as above was microinjected into the male pronuclei. Injected eggs were cultured *ex vivo* until they were implanted into the oviduct. The eggs were implanted at different sites depending on the developmental stage of the eggs; embryos in the 1-2 cell stage were implanted inside the oviduct, while embryos in the 8-cell to blastocyst stage were implanted into the ovary.

Pseudopregnancy was induced in the recipient female mice by activating corpus luteum in advance to embryo implantation. More specifically, proestrus female mice were subjected to infertile copulation with male mice that had undergone vasoligation. Due date was set as the 20th day from the 1st day when the vaginal plug was formed in the recipient. Embryos in the 1-2 cell stage were implanted under a stereomicroscope into the oviduct of the recipient mice anesthetized with Nembutal at day 1 of pseudopregnancy. About ten embryos were implanted in each side of the oviduct. When implanting into the ovary, embryos grown to molura or blastcyst *ex vivo* were implanted into the ovary of pseudopregnant recipient mice anesthetized with Nembutal. The duration of pregnancy of the recipient mice was calculated as 1 day shorter than the age of the implanted embryos. The litter size was judged by the external appearance. When five or more litters were expected, natural delivery was allowed, whereas when less than five was expected, cesarean section was performed. Litters were separated from their parents between 3-4 weeks after birth, and males and females were grown separately.

### [Example 3] Selection of transgenic mice

Genomic DNA was extracted from the tail of mice older than 4 weeks after birth using the Qiagen tissue kit (Qiagen). The DNA was used as a template for genomic PCR to amplify the transgene fragments Three pairs of primers were used for the PCR: CMV-F1 (5'-GTC GAC ATT GAT TAT TGA CTA G-3', SEQ ID NO: 9) and CMV-R1 primer (5'-CCA TAA GGT CAT GTA CTG-3', SEQ ID NO: 10); β-g1-3 (5'-CTT CTG GCG TGT GAC CGG CG-3', SEQ ID NO: 11) and hM2-2 primer (5'-ATC GAA TTC TGA GAT CAT AAT CCC TGT GGG ATG C-3', SEQ ID NO: 12); and hM8-1 (5'-TTA TTC AGT GGC AAA GTT TCT TGC CCT TGA-3', SEQ ID NO: 13) and β-globinR primer (5'-TCG AGG GAT CTT CAT AAG AGA AGA G-3', SEQ ID NO: 14), and mice in which PCR with all three primer pairs gave rise to amplified products were selected. Six selected F0 mice (3 males and 3 females) were crossed with wild-type mice (C57BL/6N Jcl) to obtain F1 progenies, from which heterozygotes were selected and crossed with each other to obtain F2 progenies.

### [Example 4] Analysis of the expression of human megsin gene (I): Northern blot analysis

The expression of human megsin gene was analyzed by northern blotting as follows. The BglII-BamHI fragment of the pBsCAG-2 vector containing the human megsin gene was labeled with radioisotope by random DNA labeling to obtain a probe. This fragment corresponds to the vicinity of the polyA signal in the vector. Total RNA (10 µg) prepared from mouse renal mesangial cells was separated on a 1% agarose gel containing 2.2 M formamide, and transferred onto a nitrocellulose membrane, which was subjected to hybridization in the Rapid Hyb solution (Amersham, Arlington Heights, IL). Then, the membrane was washed in 0.1x SSPE, 0.1% SDS at 55°C, which are highly stringent conditions. Transgenic mice A1-14 (chimera) and F1-18 (F1), and wild-type mice (nontransgenic mice) A1-11 (littermate of A1-14) and F1-19 (F1) were examined. Normal mice (C57BL/6), which were not transformed, were used as a negative control.

As shown in Figure 2, the results confirmed that the exogenous human megsin gene was expressed in the renal mesangial cells of transgenic mice (Lane 2 and 4).

### [Example 5] Production and purification of an antibody against human megsin

Polyclonal antibody against the megsin protein was produced by using as immunogen a region having a low identity with other members of the serpin family and which is hydrophilic. More specifically, a peptide with the following amino acid sequence derived from the amino acid sequence of human megsin (SEQ ID NO: 2) was selected:
Position from N-terminal: 72-86
Amino acid sequence: SQSGLQSQLKRVFSD
SEQ ID NO: 15
Peptide name: P₂.

Peptide having the amino acid sequence shown above with a C attached to the N-terminus was synthesized on the automatic synthesizer model 432A (Perkin Elmer, Foster City, CA). After purifying the synthetic peptide by reverse phase HPLC, the peptide was freeze-dried and used in a competitive experiment to confirm immunity and immunological specificity.

This synthetic peptide was bound to bovine thyroglobulin (Sigma) using N-(6-maleimidocaproyloxy)succinimide (Dojindo Molecular Technologies Inc.), was dialyzed against a 0.85% NaCl solution, and mixed well with the adjuvant (Difco) to form an emulsion, and administered subcutaneously to a rabbit. 3 weeks after priming (20 µg/rabbit), a second immunization (50 µg/rabbit) was carried out, and thereafter, four immunizations (50, 100, 200 µg/rabbit) were performed every two weeks. Freund's complete adjuvant was used for the priming, and thereafter, incomplete Freund's adjuvant was used. After 41 days and 55 days, the antibody titer of the serum was evaluated by an enzyme-linked immunosorbent assay (ELISA) to confirm that the serum obtained by blood sampling reacts with the synthetic peptide. To conduct the primary reaction, 100 µL of serially diluted antiserum was added to each well of the 96 well plate, in which 50 ng/well antigen had been immobilized. After washing, the HRP-conjugated goat anti-rabbit IgG (Cappel) was reacted in the second reaction. After washing, the coloring reaction was done using orthophenylenediamine (Wako Pure Chemical Industries, Ltd.) as the substrate and absorbance was measured at 492 nm. An increase in antibody titer was confirmed.

The polyclonal antibody against the synthetic peptide of megsin protein was purified by immunoaffinity chromatography according to a conventional method (Cell Engineering supplement "Jikken Protocol Series Kou-peptide Jikken protocol (Experimental protocol series: Anti-peptide experimental protocol)", Shujun-sha, Japan). More specifically, the method was carried out as follows: 1) an affinity column was prepared by immobilizing the synthetic peptide in FMP (2-fluoro-1-methylpyridinium toluene-4-sulfonate) activated Cellulofine (Seikagaku Corp.) ; 2) after the rabbit serum, which was prepared by immunization with megsin protein, showed increased antibody titer, it was diluted with PBS (-), and the antibody was purified by affinity using the peptide column. It was demonstrated that the obtained purified antibody is specific to the megsin protein, by confirming its reaction against megsin protein fusion protein by Western blotting.

### [Example 6] Analysis of the expression of human megsin gene (II): Immunoblot analysis

The expression of human megsin protein was analyzed by immunoblotting as follows. Kidney tissues (10 mg) were homogenized in 100 µl of 0.35 M Tris-HCl (pH 6.8) containing 10% SDS, 36% glycerol, 5% β-mercaptoethanol and 0.012% bromophenol blue and centrifuged at 5,000 g for 15 min. The proteins in the supernatant were denatured by boiling for 5 min, separated by SDS-PAGE on a 10% acrylamide gel and electrophoretically transferred to a polyvinylidene difluoride membrane (Bio Rad Laboratories, Hercules, CA). The membrane was blocked overnight at 4°C with phosphate-buffered saline (PBS) containing 0.5% Tween 20 and 2% bovine serum albumin, and incubated with rabbit anti-human megsin IgG (10 µg/ml). After washing with PBS containing 0.05% Tween 20, the membrane was incubated with 1:5,000 diluted alkaline phosphatase conjugate goat anti-rabbit IgG (Cappel, Durham, NC), followed by the development with *p*-nitro blue tetrazolium chloride and 5-bromo-4-chloro-3-indolyl-phosphate solution (Bio Rad Laboratories). As a positive control, fusion megsin protein with maltose binding protein (MBP-megsin) expressed in *E. coli* was used.

As shown in Figure 3, the present inventor confirmed that human megsin protein was expressed in the kidney of transgenic mice (Lane 3 and 5).

### [Example 7] Analysis of the expression of human megsin gene (III): Immunohistochemistry

Kidney tissues were excised from transgenic mice, and embedded in theb O. C. T. compound (Tissue Tek Miles Inc., Elkhart, IN), snap-frozen in acetone and dry ice, then sectioned at 4 µm. The frozen sections were blocked with 4% skim milk for 60 min at room temperature and subsequently incubated with 1:200 diluted fluorescein isothiocyanate (FITC)-conjugated goat anti mouse IgG, IgA, IgM or C3 antibodies (Cappel) at 4°C overnight. Then, the sections were incubated with 1:50 diluted peroxidase-conjugated swine anti-rabbit antibodies (Dako, Glostrup, Denmark) for 20 minutes at room temperature and developed with a diaminobenzidine solution containing 0.03% H₂O₂, followed by counterstaining with hematoxylin.

Micrographs in Figure 4 show immunohistochemical staining of kidney sections of transgenic mice (Microscope: Zeiss Ortholux 9902, Carl Zeiss (New York, NY)). As is obvious from the figure, megsin protein was ubiquitously expressed in the kidney of transgenic mice.

### [Example 8] Pathohistological analysis of megsin transgenic.mice (I): PAS staining and quantification of the result

F1 transgenic mice obtained in Example 3 were subjected to pathohistological analysis. Appearance and behavior of these mice were normal and indistinguishable from those of wild-type mice. No significant change was recognized by autopsy except for the kidney as described below. HE staining of tissue sections did not find any abnormalities in the major organs, except the kidney.

Each transgenic mouse kidney was stained with PAS to examine a change in the area of mesangial matrix and the number of mesangial cells. Although F1 heterozygous mice did not show any abnormality until 10 weeks after birth, they showed proliferation of cells (mainly mesangial cells) and expansion of the mesangial matrix, and developed proliferative nephritis after 36-40 weeks. PAS staining of the kidney section is shown in Figure 5. There were typical symptoms of mesangial proliferative nephritis such as expansion of glomeruli, increase in the number of mesangial cells, and expansion of the mesangial matrix.

The resultwas quantitated as follows. PAS staining photograph was scanned using a 3 CCD camera (Olympus, Tokyo, Japan) and, the size of a whole glomerulus, and the number of mesangial cells in a glomerulus were analyzed using the software Image Grabber PCI (Fuji Film, Tokyo, Japan) and Mac Aspect (Mitani, Tokyo, Japan). The glomerular size was defined by encircling outer region of the glomerular capillary tuft. Twenty consecutive glomeruli existing in the midcortex were identified and measured. Glomerular cross-sections containing less than 20 glomeruli were not utilized. In order to avoid the fluctuations of results depending on the experimenter, both the largest and smallest glomeruli in the 20 glomeruli were excluded. Eighteen glomeruli from each section were examined and averages were expressed as means ± SD. Analysis of variance (ANOVA) was utilized to evaluate the statistical significance of various differences. If a significant difference was seen, the Scheffe's t-test was used to compare results obtained from transgenic and wild-type mice.

The present inventor measured glomerular sizes and numbers of mesangial cells in 25 wild-type mice, and when the value obtained for a transgenic mouse was equal to or less than mean + 2SD of the value for a normal mouse, it was taken to be normal. Utilizing this standard, 3 out of 14 heterozygous F1 transgenic mice at 20 weeks after birth developed a change. At 40 weeks, among 19 heterozygous transgenic mice, 11 (57.9%) developed an increase in glomerular size and 12 (63.2%) developed the increase in glomerular cell number (figure 6). Altogether, 14 (73.0%) out of 19 transgenic mice examined exhibited histological glomerular abnormalities. The present inventor did not observe any sex differences in either severity or penetrance. Neither observed were any pathological changes in other organs so far examined in these lines, e.g., brain, heart, lung, spleen, liver, stomach, intestine, testis and uterus. Except for one wild-type mice that developed an expansion of the mesangial matrix at 40 weeks, no wild-type mice exhibited pathological changes.

### [Example 9] Pathohistological analysis of megsin transgenic mice (II): Observation of immune complex deposition

Kidney sections of F1 transgenic mice at 40 weeks of age when renal disorders begin to show were examined by immunofluorescent staining for the deposition of immune complexes. In the glomerular mesangium of F1 transgenic mice, deposits of immune complexes comprising immunoglobulin (IgA, IgG, and IgM) and complement were significantly increased compared with wild-type mice of the same age (Figure 7).

The same sample was examined by electron microscopy. Kidney tissue samples were immersed for 2 hr in 0.1 M sodium phosphate buffer containing 2% glutaraldehyde, postfixed in 2% osminum tetroxide, dehydrated in ethanol, and finally embedded in Epon 812 (TAAB, England). Ultrathin sections were stained with uranyl acetate and lead aceton, and analyzed by electron microscopy (JEM-1200EX, JEOL, Tokyo, Japan).

As a result, electron microscopic analysis demonstrated a small number of electron dense deposits in the mesangial area of some non-transgenic mice. In contrast, the presence of the electron dense deposits was markedly augmented in the transgenic mice: sizes of the deposits varied (100 - 1000 nm). Hypercellularity in the mesangial area of the transgenic mice was due to an increase in the number of mesangial cells, accompanied by an expansion of the mesangial matrix. Pathological findings were confined to the mesangial area. Capillary endothelium generally maintained a normal appearance, and endothelial fenestrations remained preserved. Podocytes also remained intact, maintaining normal morphologic features of foot processes. Cortical tubules showed normal appearances. Infiltration of mononuclear leukocytes or polymorphonuclear leukocytes was undetectable.

### [Example 10] Northern blot analysis of human megsin expression in a variety of tissues of the transgenic mice

Total RNA was rapidly isolated from frozen kidneys and various tissues using ISOGEN (Wako Pure Chemicals, Osaka, Japan). Twenty microgram of RNA was electrophoresed on a 1% agarose formaldehyde gel, followed by capillary transfer to a nylon membrane (GeneScreen Plus™). The blot was hybridized with ³²P-labeled human megsin transgene, and washed with 0.2x SSC containing 0.1% sodium dodecylsulfate (SDS) prior to autoradiography.

Northern blot analysis demonstrated successful overexpression of human megsin mRNA in the transgenic mice (figure 9). The transgene was expressed in a variety of tissues, especially in the kidney and the heart. Expression of human megsin cDNA in transgenic mice started at preimplantation stage and continued even after 40 weeks of age.

### [Example 11] Immunohistochemical analysis of depositions of type IV collagen and laminin

To identify the component(s) of the expanded matrix, immunohistochemical analysis of frozen sections was performed using the following antibodies (Nangaku M. et al., J. Am. Soc. Nephrol. 10: 2323-2331 (1999)). To detect type IV and type I collagen, goat anti-type IV collagen polyclonal antibody and goat anti-type I collagen polyclonal antibody (both from Southern Biotechnology, Birmingham AL) were used respectively. To detect fibronectin, rabbit anti-fibronectin antibody (Chemicon) was used.

Figure 10 shows the result of immunohistochemical staining of kidney sections with the above antibodies against the mesangial matrix components. The accumulation of type I collagen was not observed in either wild type or transgenic mice. Transgenic mice showed marked accumulation of type IV collagen and laminin in their sclerotic glomeruli. In contrast, the amount of fibronectin was much less in the transgenic mice as compared with wild type mice. Thus, it became evident that the expanded mesangial matrix comprised type IV collagen and laminin.

Although megsin expression was confined to the glomerular mesangium area under normal conditions, in the transgenic mice, it was observed in all tissues. This is thought to be due to the use of a promoter that is not tissue-type specific when introducing the gene. Nevertheless, although immunohistochemical studies reveal the presence of megsin in a host of tissues as well as in other non-mesangial areas of the kidney, harmful effects of megsin overexpression are restricted to the mesangium. This finding is of great interest as it suggests the existence of a target molecule of megsin (megsin ligand) that is specifically localized within the mesangium. In other words, it is possible that the activity of megsin requires the aid of some ligand only present in that region. Taken together, it was confirmed that overexpression of megsin in the mesangial region leads to the development of typical symptoms of mesangial proliferative nephritis.

As the above results show, human megsin transgenic mice developed progressive mesangial matrix expansion and an increase in the number of mesangial cells. Expanded matrix comprised type IV collagen and laminin. These glomerular damages were accompanied by an augmented immune complex deposition comprising immunoglobulins and complement.

Overexpression of megsin resulted in mesangial dysfunctions reflecting human disease more closely. These results suggest that transgenic mice expressing megsin gene can be used as a model to study pharmaceutical agent s capable of regulating the course of mesangial disorders as well as maintaining kidney functions. Also, use of the animal model for mesangial proliferative nephritis of the invention may provide clues to understand the pathogenesis of human glomerular disorders based on megsin function.

### Industrial Applicability

The present invention provides a transgenic animal model for mesangial proliferative nephritis, which could be achieved by the introduction of a megsin gene. The transgenic animal of the invention develops characteristic disorders such as abnormal cell proliferation and expansion of the extracellular matrix in the glomerular mesangium and endothelium in a chronically progressive manner. This strongly suggests that these mice develop a disease similar to human chronic glomerular nephritis. Thus, the animal model will be useful for the understanding of the mechanism of chronic glomerular nephritis. Such pathological findings have not been reported in conventional animal models.

Use of the animal model of the present invention will enable one to study the pathogenesis and mechanism of mesangial proliferative nephritis. It will also be useful for the development of and screening for a therapeutic agent for mesangial proliferative nephritis as well as testing the agents.

The transgenic animal model for mesangial proliferative nephritis of this invention will easily provide a homogenous population in a large quantity, which facilitates highly reliable experiments. Moreover, the animal model for mesangial proliferative nephritis develops typical symptoms of human mesangial proliferative nephritis such as the expansion of mesangial matrix, proliferation of mesangial cells, and deposition of immune complexes. Thus, the present invention that provides an animal model that closely reflects human disease has a great significance.

## Claims

1. An animal model for mesangial proliferative nephritis, wherein the animal model is prepared using a non-human transgenic animal that expresses an exogenous DNA encoding human megsin, or. a functionally equivalent protein thereof, in its renal mesangial cells.

2. The animal model for mesangial proliferative nephritis according to claim 1, wherein the DNA encoding human megsin comprises the coding region of the nucleotide sequence of SEQ ID NO: 1.

3. The animal model for mesangial proliferative nephritis according to claim 1, wherein the non-human animal is a mouse.

4. A transgene for generating an animal model for mesangial proliferative nephritis, comprising a DNA encoding human megsin, or a functionally equivalent protein thereof, and a promoter capable of induces expressing expression of the DNA in renal mesangial cells of the animal.

5. A method for preparing an animal model for mesangial proliferative nephritis, comprising the steps of:
(a) introducing the transgene according to claim 4 into a fertilized egg of an animal, and,
(b) selecting the transgenic animal that carries the introduced exogenous gene.

6. The method for preparing an animal model of mesangial proliferative nephritis according to claim 5, further comprising the following steps of:
(c) crossing the animal selected in step (b) with a wild-type animal to obtain F1 progenies comprising exogenous heterozygous genes, and,
(d) crossing the F1 progenies obtained in step (c) with each other to obtain F2 progenies comprising exogenous homozygous genes.

7. A method of predicting the therapeutic effect of a candidate compound on mesangial proliferative nephritis, comprising the following steps of:
(a) administrating a candidate compound to the animal model of mesangial proliferative nephritis according to claim 1, and
(b) observing a change in a symptom of nephritis in the animal model.

8. A method of screening for a therapeutic compound for. mesangial proliferative nephritis, comprising the following steps of:
(a) administrating a candidate compound to the animal model for mesangial proliferative nephritis according to claim 1,
(b) observing a change in a symptom of nephritis in the animal model, and,
(c) selecting the candidate compound that ameliorates the symptom of mesangial proliferative nephritis.

9. A pharmaceutical composition for treatment and/or prevention of mesangial proliferative nephritis, wherein the pharmaceutical composition comprises a compound obtained by the method of screening according to claim 8 as the active ingredient.


SEQUENCE LISTING

<110> MIYATA, Toshio
      KUROKAWA, Kiyoshi

<120> ANIMAL MODEL FOR MESANGIAL PROLIFERATIVE NEPHRITIS

<130> KRK-108PCT

<140>
<141>

<150> JP 1999-285736
<151> 1999-10-06

<160> 15

<170> PatentIn Ver. 2.0

<210> 1
<211> 1143
<212> DNA
<213> Homo sapiens

<220>
<221> CDS
<222> (1)..(1140)

<400> 1
atg gcc tcc ctt gct gca gca aat gca gag ttt tgc ttc aac ctg ttc 48
Met Ala Ser Leu Ala Ala Ala Asn Ala Glu Phe Cys Phe Asn Leu Phe
  1               5                  10                  15

aga gag atg gat gac aat caa gga aat gga aat gtg ttc ttt tcc tct 96
Arg Glu Met Asp Asp Asn Gln Gly Asn Gly Asn Val Phe Phe Ser Ser
             20                  25                  30

ctg agc ctc ttc gct gcc ctg gcc ctg gtc cgc ttg ggc gct caa gat 144
Leu Ser Leu Phe Ala Ala Leu Ala Leu Val Arg Leu Gly Ala Gln Asp
         35                  40                  45

gac tcc ctc tct cag att gat aag ttg ctt cat gtt aac act gcc tca 192
Asp Ser Leu Ser Gln Ile Asp Lys Leu Leu His Val Asn Thr Ala Ser
     50                  55                  60

gga tat gga aac tct tct aat agt cag tca ggg ctc cag tct caa ctg 240
Gly Tyr Gly Asn Ser Ser Asn Ser Gln Ser Gly Leu Gln Ser Gln Leu

65                  70                  75                  80

aaa aga gtt ttt tct gat ata aat gca tcc cac aag gat tat gat ctc     288
Lys Arg Val Phe Ser Asp Ile Asn Ala Ser His Lys Asp Tyr Asp Leu
                85                  90                  95

agc att gtg aat ggg ctt ttt gct gaa aaa gtg tat ggc ttt cat aag     336
Ser Ile Val Asn Gly Leu Phe Ala Glu Lys Val Tyr Gly Phe His Lys
            100                 105                 110

gac tac att gag tgt gcc gaa aaa tta tac gat gcc aaa gtg gag cga     384
Asp Tyr Ile Glu Cys Ala Glu Lys Leu Tyr Asp Ala Lys Val Glu Arg
        115                 120                 125

gtt gac ttt acg aat cat tta gaa gac act aga cgt aat att aat aag     432
Val Asp Phe Thr Asn His Leu Glu Asp Thr Arg Arg Asn Ile Asn Lys
    130                 135                 140

tgg gtt gaa aat gaa aca cat ggc aaa atc aag aac gtg att ggt gaa     480
Trp Val Glu Asn Glu Thr His Gly Lys Ile Lys Asn Val Ile Gly Glu
145                 150                 155                 160

ggt ggc ata agc tca tct gct gta atg gtg ctg gtg aat gct gtg tac     528
Gly Gly Ile Ser Ser Ser Ala Val Met Val Leu Val Asn Ala Val Tyr
                165                 170                 175

ttc aaa ggc aag tgg caa tca gcc ttc acc aag agc gaa acc ata aat     576
Phe Lys Gly Lys Trp Gln Ser Ala Phe Thr Lys Ser Glu Thr Ile Asn
            180                 185                 190

tgc cat ttc aaa tct ccc aag tgc tct ggg aag gca gtc gcc atg atg     624
Cys His Phe Lys Ser Pro Lys Cys Ser Gly Lys Ala Val Ala Met Met
        195                 200                 205

cat cag gaa cgg aag ttc aat ttg tct gtt att gag gac cca tca atg     672
His Gln Glu Arg Lys Phe Asn Leu Ser Val Ile Glu Asp Pro Ser Met
    210                 215                 220

aag att ctt gag ctc aga tac aat ggt ggc ata aac atg tac gtt ctg     720
Lys Ile Leu Glu Leu Arg Tyr Asn Gly Gly Ile Asn Met Tyr Val Leu
225                 230                 235                 240

ctg cct gag aat gac ctc tct gaa att gaa aac aaa ctg acc ttt cag     768
Leu Pro Glu Asn Asp Leu Ser Glu Ile Glu Asn Lys Leu Thr Phe Gln
                245                 250                 255

aat cta atg gaa tgg acc aat cca agg cga atg acc tct aag tat gtt     816
Asn Leu Met Glu Trp Thr Asn Pro Arg Arg Met Thr Ser Lys Tyr Val

            260                 265                 270

gag gta ttt ttt cct cag ttc aag ata gag aag aat tat gaa atg aaa 864
Glu Val Phe Phe Pro Gln Phe Lys Ile Glu Lys Asn Tyr Glu Met Lys
        275                 280                 285

caa tat ttg aga gcc cta ggg ctg aaa gat atc ttt gat gaa tcc aaa 912
Gln Tyr Leu Arg Ala Leu Gly Leu Lys Asp Ile Phe Asp Glu Ser Lys
    290                 295                 300

gca gat ctc tct ggg att gct tcg ggg ggt cgt ctg tat ata tca agg 960
Ala Asp Leu Ser Gly Ile Ala Ser Gly Gly Arg Leu Tyr Ile Ser Arg
305                 310                 315                 320

atg atg cac aaa tct tac ata gag gtc act gag gag ggc acc gag gct 1008
Met Met His Lys Ser Tyr Ile Glu Val Thr Glu Glu Gly Thr Glu Ala
                325                 330                 335

act gct gcc aca gga agt aat att gta gaa aag caa ctc cct cag tcc 1056
Thr Ala Ala Thr Gly Ser Asn Ile Val Glu Lys Gln Leu Pro Gln Ser
            340                 345                 350

acg ctg ttt aga gct gac cac cca ttc cta ttt gtt atc agg aag gat 1104
Thr Leu Phe Arg Ala Asp His Pro Phe Leu Phe Val Ile Arg Lys Asp
        355                 360                 365

gac atc atc tta ttc agt ggc aaa gtt tct tgc cct tga 1143
Asp Ile Ile Leu Phe Ser Gly Lys Val Ser Cys Pro
    370                 375                 380

<210> 2
<211> 380
<212> PRT
<213> Homo sapiens

<400> 2
Met Ala Ser Leu Ala Ala Ala Asn Ala Glu Phe Cys Phe Asn Leu Phe
1               5                   10                  15

Arg Glu Met Asp Asp Asn Gln Gly Asn Gly Asn Val Phe Phe Ser Ser
            20                  25                  30

Leu Ser Leu Phe Ala Ala Leu Ala Leu Val Arg Leu Gly Ala Gln Asp
        35                  40                  45

Asp Ser Leu Ser Gln Ile Asp Lys Leu Leu His Val Asn Thr Ala Ser
    50                  55                  60

Gly Tyr Gly Asn Ser Ser Asn Ser Gln Ser Gly Leu Gln Ser Gln Leu
65                  70                  75                  80

Lys Arg Val Phe Ser Asp Ile Asn Ala Ser His Lys Asp Tyr Asp Leu
                85                  90                  95

Ser Ile Val Asn Gly Leu Phe Ala Glu Lys Val Tyr Gly Phe His Lys
            100                 105                 110

Asp Tyr Ile Glu Cys Ala Glu Lys Leu Tyr Asp Ala Lys Val Glu Arg
        115                 120                 125

Val Asp Phe Thr Asn His Leu Glu Asp Thr Arg Arg Asn Ile Asn Lys
    130                 135                 140

Trp Val Glu Asn Glu Thr His Gly Lys Ile Lys Asn Val Ile Gly Glu
145                 150                 155                 160

Gly Gly Ile Ser Ser Ser Ala Val Met Val Leu Val Asn Ala Val Tyr
                165                 170                 175

Phe Lys Gly Lys Trp Gln Ser Ala Phe Thr Lys Ser Glu Thr Ile Asn
            180                 185                 190

Cys His Phe Lys Ser Pro Lys Cys Ser Gly Lys Ala Val Ala Met Met
        195                 200                 205

His Gln Glu Arg Lys Phe Asn Leu Ser Val Ile Glu Asp Pro Ser Met
    210                 215                 220

Lys Ile Leu Glu Leu Arg Tyr Asn Gly Gly Ile Asn Met Tyr Val Leu
225                 230                 235                 240

Leu Pro Glu Asn Asp Leu Ser Glu Ile Glu Asn Lys Leu Thr Phe Gln
                245                 250                 255

Asn Leu Met Glu Trp Thr Asn Pro Arg Arg Met Thr Ser Lys Tyr Val
            260                 265                 270

Glu Val Phe Phe Pro Gln Phe Lys Ile Glu Lys Asn Tyr Glu Met Lys
        275                 280                 285

Gln Tyr Leu Arg Ala Leu Gly Leu Lys Asp Ile Phe Asp Glu Ser Lys
    290                 295                 300

Ala Asp Leu Ser Gly Ile Ala Ser Gly Gly Arg Leu Tyr Ile Ser Arg
305                 310                 315                 320

Met Met His Lys Ser Tyr Ile Glu Val Thr Glu Glu Gly Thr Glu Ala
                325                 330                 335

Thr Ala Ala Thr Gly Ser Asn Ile Val Glu Lys Gln Leu Pro Gln Ser
            340                 345                 350

Thr Leu Phe Arg Ala Asp His Pro Phe Leu Phe Val Ile Arg Lys Asp
        355                 360                 365

Asp Ile Ile Leu Phe Ser Gly Lys Val Ser Cys Pro
    370                 375                 380

<210> 3
<211> 1229
<212> DNA
<213> Rattus norvegicus

<220>
<221> CDS
<222> (8)..(1147)

<400> 3
tttcaaa atg gcc tcc ctt gct gca gca aat gca gaa ttt ggc ttc gac      49
        Met Ala Ser Leu Ala Ala Ala Asn Ala Glu Phe Gly Phe Asp
          1               5                  10

tta ttc aga gag atg gat agt agt caa gga aac gga aat gta ttc ttc      97
Leu Phe Arg Glu Met Asp Ser Ser Gln Gly Asn Gly Asn Val Phe Phe
 15                  20                  25                  30

tct tcc ctg agc atc ttc act gcc ctg agc cta atc cgt ttg ggt gct     145
Ser Ser Leu Ser Ile Phe Thr Ala Leu Ser Leu Ile Arg Leu Gly Ala
                 35                  40                  45

cga ggt gac tgt nnn cgt cag att gac aag gcc ctg cac ttt atc tcc     193
Arg Gly Asp Cys Xaa Arg Gln Ile Asp Lys Ala Leu His Phe Ile Ser
             50                  55                  60

cca tca aga caa ggg aat tca tcg aac agt cag cta gga ctg caa tat     241
Pro Ser Arg Gln Gly Asn Ser Ser Asn Ser Gln Leu Gly Leu Gln Tyr
         65                  70                  75

caa ttg aaa aga gtt ctt gct gac ata aac tca tct cat aag gat nnn     289
Gln Leu Lys Arg Val Leu Ala Asp Ile Asn Ser Ser His Lys Asp Xaa
     80                  85                  90

aaa ctc agc att gcc aat gga gtt ttt gca gag aaa gta ttt gat ttt 337
Lys Leu Ser Ile Ala Asn Gly Val Phe Ala Glu Lys Val Phe Asp Phe
95                  100                 105                 110

cat aag agc tat atg gag tgt gct gaa aac tta tac aat gct aaa gtg 385
His Lys Ser Tyr Met Glu Cys Ala Glu Asn Leu Tyr Asn Ala Lys Val
                115                 120                 125

gaa aga gtt gat ttt aca aat gat ata caa gaa acc aga ttt aaa att 433
Glu Arg Val Asp Phe Thr Asn Asp Ile Gln Glu Thr Arg Phe Lys Ile
            130                 135                 140

aat aaa tgg att gaa aat gaa aca cat ggc aaa atc aag aag gtg ttg 481
Asn Lys Trp Ile Glu Asn Glu Thr His Gly Lys Ile Lys Lys Val Leu
        145                 150                 155

ggg gac agc agc ctc agc tca tca gct gtc atg gtg cta gtg aat gct 529
Gly Asp Ser Ser Leu Ser Ser Ser Ala Val Met Val Leu Val Asn Ala
    160                 165                 170

gtt tac ttc aaa ggc aag tgg aaa tcg gcc ttc acc aag agt gat acc 577
Val Tyr Phe Lys Gly Lys Trp Lys Ser Ala Phe Thr Lys Ser Asp Thr
175                 180                 185                 190

ctc agt tgc cat ttc agg tct ccc agc ggt cct gga aaa gca gtt aat 625
Leu Ser Cys His Phe Arg Ser Pro Ser Gly Pro Gly Lys Ala Val Asn
                195                 200                 205

atg atg cat caa gaa cgg agg ttc aat ttg tct acc att cag gag cca 673
Met Met His Gln Glu Arg Arg Phe Asn Leu Ser Thr Ile Gln Glu Pro
            210                 215                 220

cca atg cag att ctt gag cta caa tat cat ggt ggc ata agc atg tac 721
Pro Met Gln Ile Leu Glu Leu Gln Tyr His Gly Gly Ile Ser Met Tyr
        225                 230                 235

atc atg ttg ccc gag gat gac cta tcc gaa att gaa agc aag ctg agt 769
Ile Met Leu Pro Glu Asp Asp Leu Ser Glu Ile Glu Ser Lys Leu Ser
    240                 245                 250

ttc cag aat cta atg gac tgg aca aat agc agg aag atg aaa tct cag 817
Phe Gln Asn Leu Met Asp Trp Thr Asn Ser Arg Lys Met Lys Ser Gln
255                 260                 265                 270

tat gtg aat gtg ttt ctc ccc cag ttc aag ata gag aaa gat tat gaa 865
Tyr Val Asn Val Phe Leu Pro Gln Phe Lys Ile Glu Lys Asp Tyr Glu
                275                 280                 285

atg agg agc cac ttg aaa tct gta ggc ttg gaa gac atc ttt gtt gag 913
Met Arg Ser His Leu Lys Ser Val Gly Leu Glu Asp Ile Phe Val Glu
            290                 295                 300

tcc agg gct gat ctg tct gga att gcc tct gga ggt cgt ctc tat gta 961
Ser Arg Ala Asp Leu Ser Gly Ile Ala Ser Gly Gly Arg Leu Tyr Val
        305                 310                 315

tca aag cta atg cac aag tcc ctc ata gag gtc tca gaa gaa ggc acc 1009
Ser Lys Leu Met His Lys Ser Leu Ile Glu Val Ser Glu Glu Gly Thr
    320                 325                 330

gag gca act gct gcc aca gaa agt aac atc gtt gaa aag cta ctt cct 1057
Glu Ala Thr Ala Ala Thr Glu Ser Asn Ile Val Glu Lys Leu Leu Pro
335                 340                 345                 350

gaa tcc acg gtg ttc aga gct gac cgc ccc ttt ctg ttt gtc att agg 1105
Glu Ser Thr Val Phe Arg Ala Asp Arg Pro Phe Leu Phe Val Ile Arg
                355                 360                 365

aag aat ggc atc atc tta ttt act ggc aaa gtc tcg tgt cct 1147
Lys Asn Gly Ile Ile Leu Phe Thr Gly Lys Val Ser Cys Pro
            370                 375                 380

tgaaattcta tttggttttc catacactaa caggcatgaa gaaacatcat aagtgaatag 1207

aattgtaatt ggaagtacat gg 1229

<210> 4
<211> 380
<212> PRT
<213> Rattus norvegicus

<400> 4
Met Ala Ser Leu Ala Ala Ala Asn Ala Glu Phe Gly Phe Asp Leu Phe
1               5                   10                  15

Arg Glu Met Asp Ser Ser Gln Gly Asn Gly Asn Val Phe Phe Ser Ser
            20                  25                  30

Leu Ser Ile Phe Thr Ala Leu Ser Leu Ile Arg Leu Gly Ala Arg Gly
        35                  40                  45

Asp Cys Xaa Arg Gln Ile Asp Lys Ala Leu His Phe Ile Ser Pro Ser
    50                  55                  60

Arg Gln Gly Asn Ser Ser Asn Ser Gln Leu Gly Leu Gln Tyr Gln Leu

65                  70                  75                  80

Lys Arg Val Leu Ala Asp Ile Asn Ser Ser His Lys Asp Xaa Lys Leu
                85                  90                  95

Ser Ile Ala Asn Gly Val Phe Ala Glu Lys Val Phe Asp Phe His Lys
            100                 105                 110

Ser Tyr Met Glu Cys Ala Glu Asn Leu Tyr Asn Ala Lys Val Glu Arg
        115                 120                 125

Val Asp Phe Thr Asn Asp Ile Gln Glu Thr Arg Phe Lys Ile Asn Lys
    130                 135                 140

Trp Ile Glu Asn Glu Thr His Gly Lys Ile Lys Lys Val Leu Gly Asp
145                 150                 155                 160

Ser Ser Leu Ser Ser Ser Ala Val Met Val Leu Val Asn Ala Val Tyr
                165                 170                 175

Phe Lys Gly Lys Trp Lys Ser Ala Phe Thr Lys Ser Asp Thr Leu Ser
            180                 185                 190

Cys His Phe Arg Ser Pro Ser Gly Pro Gly Lys Ala Val Asn Met Met
        195                 200                 205

His Gln Glu Arg Arg Phe Asn Leu Ser Thr Ile Gln Glu Pro Pro Met
    210                 215                 220

Gln Ile Leu Glu Leu Gln Tyr His Gly Gly Ile Ser Met Tyr Ile Met
225                 230                 235                 240

Leu Pro Glu Asp Asp Leu Ser Glu Ile Glu Ser Lys Leu Ser Phe Gln
                245                 250                 255

Asn Leu Met Asp Trp Thr Asn Ser Arg Lys Met Lys Ser Gln Tyr Val
            260                 265                 270

Asn Val Phe Leu Pro Gln Phe Lys Ile Glu Lys Asp Tyr Glu Met Arg
        275                 280                 285

Ser His Leu Lys Ser Val Gly Leu Glu Asp Ile Phe Val Glu Ser Arg
    290                 295                 300

Ala Asp Leu Ser Gly Ile Ala Ser Gly Gly Arg Leu Tyr Val Ser Lys
305                 310                 315                 320

Leu Met His Lys Ser Leu Ile Glu Val Ser Glu Glu Gly Thr Glu Ala

                325                 330                 335

Thr Ala Ala Thr Glu Ser Asn Ile Val Glu Lys Leu Leu Pro Glu Ser
            340                 345                 350

Thr Val Phe Arg Ala Asp Arg Pro Phe Leu Phe Val Ile Arg Lys Asn
        355                 360                 365

Gly Ile Ile Leu Phe Thr Gly Lys Val Ser Cys Pro
    370                 375                 380

<210> 5
<211> 1147
<212> DNA
<213> Mus musculus

<220>
<221> CDS
<222> (1)..(1104)

<400> 5
ttc gac tta ttc aga gag atg gat agt agc caa gga aat gga aat gta 48
Phe Asp Leu Phe Arg Glu Met Asp Ser Ser Gln Gly Asn Gly Asn Val
1               5                   10                  15

ttc ttc tct tcc ctg agc atc ttc act gcc ctg acc cta atc cgt ctg 96
Phe Phe Ser Ser Leu Ser Ile Phe Thr Ala Leu Thr Leu Ile Arg Leu
            20                  25                  30

ggt gct cga ggt gac tgt gca cgt cag att gac aag gca ctg cac ttt 144
Gly Ala Arg Gly Asp Cys Ala Arg Gln Ile Asp Lys Ala Leu His Phe
        35                  40                  45

aac ata cca tca aga caa gga aac tca tct aat aat cag cca gga ctt 192
Asn Ile Pro Ser Arg Gln Gly Asn Ser Ser Asn Asn Gln Pro Gly Leu
    50                  55                  60

cag tat caa ttg aaa aga gtt ctt gct gac ata aac tca tct cat aag 240
Gln Tyr Gln Leu Lys Arg Val Leu Ala Asp Ile Asn Ser Ser His Lys
65                  70                  75                  80

gat tat gaa ctc agc att gcc act gga gtt ttt gca gaa aaa gtc tat 288
Asp Tyr Glu Leu Ser Ile Ala Thr Gly Val Phe Ala Glu Lys Val Tyr
                85                  90                  95

gac ttt cat aag aac tac att gag tgt gct gaa aac tta tac aat gct 336
Asp Phe His Lys Asn Tyr Ile Glu Cys Ala Glu Asn Leu Tyr Asn Ala

            100                 105                 110

aaa gtg gaa aga gtt gac ttc aca aat gat gta caa gat acc aga ttt 384
Lys Val Glu Arg Val Asp Phe Thr Asn Asp Val Gln Asp Thr Arg Phe
        115                 120                 125

aaa att aat aaa tgg att gaa aat gag aca cat gga aag atc aag aag 432
Lys Ile Asn Lys Trp Ile Glu Asn Glu Thr His Gly Lys Ile Lys Lys
    130                 135                 140

gtg ttg ggc gac agc agc ctc agc tcg tcg gct gtc atg gtg ctg gtg 480
Val Leu Gly Asp Ser Ser Leu Ser Ser Ser Ala Val Met Val Leu Val
145                 150                 155                 160

aac gct gtt tac ttc aaa ggc aaa tgg aaa tcg gcc ttc acc aag act 528
Asn Ala Val Tyr Phe Lys Gly Lys Trp Lys Ser Ala Phe Thr Lys Thr
                165                 170                 175

gat acc ctc agt tgc cgt ttt agg tct ccc acg tgt cct gga aaa gta 576
Asp Thr Leu Ser Cys Arg Phe Arg Ser Pro Thr Cys Pro Gly Lys Val
            180                 185                 190

gtt aat atg atg cat caa gaa cgg cgg ttc aat ttg tct acc att cag 624
Val Asn Met Met His Gln Glu Arg Arg Phe Asn Leu Ser Thr Ile Gln
        195                 200                 205

cag cca cca atg cag gtt ctt gag ctc caa tat cat ggt ggc ata agc 672
Gln Pro Pro Met Gln Val Leu Glu Leu Gln Tyr His Gly Gly Ile Ser
    210                 215                 220

atg tac att atg ctg cct gag gat ggc cta tgt gaa att gaa agc aag 720
Met Tyr Ile Met Leu Pro Glu Asp Gly Leu Cys Glu Ile Glu Ser Lys
225                 230                 235                 240

ctg agt ttc cag aat ctg atg gac tgg acc aat agg agg aaa atg aaa 768
Leu Ser Phe Gln Asn Leu Met Asp Trp Thr Asn Arg Arg Lys Met Lys
                245                 250                 255

tct cag tat gtg aac gtg ttt ctc ccc cag ttc aag ata gag aag aat 816
Ser Gln Tyr Val Asn Val Phe Leu Pro Gln Phe Lys Ile Glu Lys Asn
            260                 265                 270

tat gaa atg acg cac cac ttg aaa tcc tta ggc ttg aaa gat atc ttt 864
Tyr Glu Met Thr His His Leu Lys Ser Leu Gly Leu Lys Asp Ile Phe
        275                 280                 285

gat gag tcc agt gca gat ctc tct gga att gcc tct gga ggt cgt ctc 912
Asp Glu Ser Ser Ala Asp Leu Ser Gly Ile Ala Ser Gly Gly Arg Leu

290                    295                    300

tac gta tca aag cta atg cac aag tca ttc ata gag gtc tca gag gag    960
Tyr Val Ser Lys Leu Met His Lys Ser Phe Ile Glu Val Ser Glu Glu
305                 310                 315                 320

ggc act gaa gcc act gct gcc aca gaa aat aac att gtt gaa aag cag    1008
Gly Thr Glu Ala Thr Ala Ala Thr Glu Asn Asn Ile Val Glu Lys Gln
                325                 330                 335

ctt cct gag tcc aca gtg ttc aga gcc gac cgc ccc ttt ctg ttt gtc    1056
Leu Pro Glu Ser Thr Val Phe Arg Ala Asp Arg Pro Phe Leu Phe Val
            340                 345                 350

atc aag aag aat gac atc atc tta ttt act ggc aaa gtc tct tgt cct    1104
Ile Lys Lys Asn Asp Ile Ile Leu Phe Thr Gly Lys Val Ser Cys Pro
        355                 360                 365

tgaaattcga tttggtttcc tatacagtaa caggcatcaa gaa                    1147

<210> 6
<211> 368
<212> PRT
<213> Mus musculus

<400> 6
Phe Asp Leu Phe Arg Glu Met Asp Ser Ser Gln Gly Asn Gly Asn Val
1               5                   10                  15

Phe Phe Ser Ser Leu Ser Ile Phe Thr Ala Leu Thr Leu Ile Arg Leu
            20                  25                  30

Gly Ala Arg Gly Asp Cys Ala Arg Gln Ile Asp Lys Ala Leu His Phe
        35                  40                  45

Asn Ile Pro Ser Arg Gln Gly Asn Ser Ser Asn Asn Gln Pro Gly Leu
    50                  55                  60

Gln Tyr Gln Leu Lys Arg Val Leu Ala Asp Ile Asn Ser Ser His Lys
65                  70                  75                  80

Asp Tyr Glu Leu Ser Ile Ala Thr Gly Val Phe Ala Glu Lys Val Tyr
                85                  90                  95

Asp Phe His Lys Asn Tyr Ile Glu Cys Ala Glu Asn Leu Tyr Asn Ala
            100                 105                 110

Lys Val Glu Arg Val Asp Phe Thr Asn Asp Val Gln Asp Thr Arg Phe
        115                 120                 125

Lys Ile Asn Lys Trp Ile Glu Asn Glu Thr His Gly Lys Ile Lys Lys
    130                 135                 140

Val Leu Gly Asp Ser Ser Leu Ser Ser Ser Ala Val Met Val Leu Val
145                 150                 155                 160

Asn Ala Val Tyr Phe Lys Gly Lys Trp Lys Ser Ala Phe Thr Lys Thr
                165                 170                 175

Asp Thr Leu Ser Cys Arg Phe Arg Ser Pro Thr Cys Pro Gly Lys Val
            180                 185                 190

Val Asn Met Met His Gln Glu Arg Arg Phe Asn Leu Ser Thr Ile Gln
        195                 200                 205

Gln Pro Pro Met Gln Val Leu Glu Leu Gln Tyr His Gly Gly Ile Ser
    210                 215                 220

Met Tyr Ile Met Leu Pro Glu Asp Gly Leu Cys Glu Ile Glu Ser Lys
225                 230                 235                 240

Leu Ser Phe Gln Asn Leu Met Asp Trp Thr Asn Arg Arg Lys Met Lys
                245                 250                 255

Ser Gln Tyr Val Asn Val Phe Leu Pro Gln Phe Lys Ile Glu Lys Asn
            260                 265                 270

Tyr Glu Met Thr His His Leu Lys Ser Leu Gly Leu Lys Asp Ile Phe
        275                 280                 285

Asp Glu Ser Ser Ala Asp Leu Ser Gly Ile Ala Ser Gly Gly Arg Leu
    290                 295                 300

Tyr Val Ser Lys Leu Met His Lys Ser Phe Ile Glu Val Ser Glu Glu
305                 310                 315                 320

Gly Thr Glu Ala Thr Ala Ala Thr Glu Asn Asn Ile Val Glu Lys Gln
                325                 330                 335

Leu Pro Glu Ser Thr Val Phe Arg Ala Asp Arg Pro Phe Leu Phe Val
            340                 345                 350

Ile Lys Lys Asn Asp Ile Ile Leu Phe Thr Gly Lys Val Ser Cys Pro
        355                 360                 365

<210> 7
<211> 44
<212> DNA
<213> Artificial Sequence

<220>
<223> Description of Artificial Sequence:an artificially
      synthesized primer sequence

<400> 7
atggatccgc cgccatggcc tcccttgctg cagcaaatgc agag                44


<210> 8
<211> 26
<212> DNA
<213> Artificial Sequence

<220>
<223> Description of Artificial Sequence:an artificially
      synthesized primer sequence

<400> 8
tatcctgagg cagtgttaac atgaag                                    26


<210> 9
<211> 22
<212> DNA
<213> Artificial Sequence

<220>
<223> Description of Artificial Sequence:an artificially
      synthesized primer sequence

<400> 9
gtcgacattg attattgact ag                                        22


<210> 10
<211> 18
<212> DNA
<213> Artificial Sequence

<220>
<223> Description of Artificial Sequence:an artificially
      synthesized primer sequence

<400> 10
ccataaggtc atgtactg                                                    18


<210> 11
<211> 20
<212> DNA
<213> Artificial Sequence

<220>
<223> Description of Artificial Sequence:an artificially
      synthesized primer sequence

<400> 11
cttctggcgt gtgaccggcg                                                  20


<210> 12
<211> 34
<212> DNA
<213> Artificial Sequence

<220>
<223> Description of Artificial Sequence:an artificially
      synthesized primer sequence

<400> 12
atcgaattct gagatcataa tccctgtggg atgc                                  34


<210> 13
<211> 30
<212> DNA
<213> Artificial Sequence

<220>
<223> Description of Artificial Sequence:an artificially
      synthesized primer sequence

<400> 13
ttattcagtg gcaaagtttc ttgcccttga                                       30


<210> 14
<211> 25
<212> DNA
<213> Artificial Sequence

<220>
<223> Description of Artificial Sequence:an artificially
      synthesized primer sequence

<400> 14
tcgagggatc ttcataagag aagag                                          25


<210> 15
<211> 15
<212> PRT
<213> Artificial Sequence

<220>
<223> Description of Artificial Sequence:Artificially
      synthesized domain peptide of human megsin

<400> 12
Ser Gln Ser Gly Leu Gln Ser Gln Leu Lys Arg Val Phe Ser Asp
 1               5                  10                  15